# DEMANDE DE BREVET EUROPEEN

(11) **EP 4 686 483 A1**
(43) Date de publication de la demande: **04.02.2026**
(21) Numéro de dépôt: 25193042.6
(22) Date de dépôt: 31.07.2025
(51) Int. Cl.: A61L 2/10, A61L 2/26

(54) **ENCEINTE DE DÉSINFECTION ET PROCÉDÉ DE DÉSINFECTION ASSOCIÉ**

(30) Priorité: 31.07.2024 FR 2408460
(71) Demandeur: Germitec, 33000 Bordeaux (FR)
(72) Inventeur: POULON, Fanny, 91440 BURES SUR YVETTE (FR); DALLARD, Jérémy, 33400 TALENCE (FR); BERTHIAS, Maxime, 33750 CADARSAC (FR); HAMDOU, Aimrane, 33800 BORDEAUX (FR)
(74) Mandataire: Lavoix

(57) **Abrégé**

L'invention concerne un enceinte de désinfection (10) d'un instrument médical comprenant :
- un volume de désinfection (24) délimité par au moins une paroi (22A-E),
- un dispositif de génération de rayonnements UV-C (26) configuré pour générer des rayonnements UV-C dans le volume de désinfection (24), et
- un support (28) configuré pour maintenir l'instrument médical en entier à l'intérieur du volume de désinfection (24), le support (28) étant transparent aux rayonnements UV-C.

## Description

La présente invention concerne une enceinte de désinfection d'un instrument médical et un procédé de désinfection associé.

Afin d'éviter toutes contaminations, il convient de désinfecter un instrument médical entre deux utilisations.

A cet effet, il est connu de désinfecter des instruments médicaux dans des enceintes de désinfection définissant un volume de désinfection fermé. De telles enceintes comprennent par exemple un dispositif de génération de rayonnements UV (Ultra-violet) permettant notamment d'effectuer une désinfection de ces instruments médicaux, et plus particulièrement une désinfection haut-niveau.

Les instruments médicaux, tels que des sondes médicales par exemple, comprennent typiquement au moins une partie active et, de manière préférentielle, également au moins une partie de raccordement sous forme de câble.

Il est connu de suspendre ces instruments médicaux dans l'enceinte de désinfection, par exemple à l'aide d'une potence de suspension comprenant par exemple une molette ou une bague adaptée pour maintenir une portion de la partie de raccordement.

Généralement, dans de telles enceintes de désinfection, au moins ladite portion de la partie de raccordement n'est pas désinfectée puisqu'elle se trouve à l'extérieur du volume de désinfection.

Une telle désinfection n'est donc pas entièrement satisfaisante, car il existe un risque de contamination au niveau de cette zone non-désinfectée.

Le but de l'invention est alors de proposer une enceinte de désinfection permettant une désinfection améliorée de l'instrument médical.

A cet effet, l'invention a pour objet une enceinte de désinfection d'un instrument médical comprenant :
- un volume de désinfection délimité par au moins une paroi,
- un dispositif de génération de rayonnements UV-C configuré pour générer des rayonnements UV-C dans le volume de désinfection, et
- un support configuré pour maintenir l'instrument médical en entier à l'intérieur du volume de désinfection, le support étant transparent aux rayonnements UV-C.

Grâce au support, l'instrument médical est maintenu en entier dans le volume de désinfection, ce qui évite qu'une partie de l'instrument ne soit pas désinfectée.

Par ailleurs, la caractéristique selon laquelle le support est transparent aux rayonnements UV-C garantit que même les portions de l'instrument médical qui sont directement maintenues par le support, notamment qui sont en contact avec celui-ci, soient atteintes par les rayonnements UV-C, et donc désinfectées.

Suivant d'autres aspects avantageux de l'invention, l'enceinte de désinfection comprend une ou plusieurs des caractéristiques suivantes, prises isolément ou suivant toutes les combinaisons techniquement possibles :
- Le support a une transmittance aux rayonnements UV-C supérieure à 30%, et de préférence supérieure à 50%.
- Le support comprend au moins une paire d'éléments comprenant un premier élément et un deuxième élément, l'instrument médical étant destiné à être maintenu par le premier et le deuxième éléments.
- L'au moins une paroi comprend au moins une première zone et une deuxième zone différente de la première zone, le premier élément étant fixé à la première zone et le deuxième élément étant fixé à la deuxième zone.
- Le support comprend deux paires d'éléments.
- Le premier et/ou le deuxième élément du ou de chaque paire d'éléments comprend une plaque comprenant une ouverture latérale d'insertion de sorte à permettre l'insertion d'une portion de l'instrument médical.
- L'au moins une paroi comprend un fond, le support comprenant un troisième élément fixé au fond, l'instrument médical étant destiné à être maintenu par le troisième élément.
- Le support est fabriqué en quartz, de préférence du quartz synthétique.

L'invention concerne également un procédé de désinfection d'un instrument médical comprenant les étapes suivantes :
a. fourniture d'une enceinte telle que décrite ci-dessus, et
b. positionnement de l'instrument médical en entier à l'intérieur du volume de désinfection, l'instrument médical étant maintenu par le support.

Avantageusement, l'instrument médical comprend une poignée et de préférence au moins un connecteur, la poignée de l'instrument médical étant maintenue par l'un des premiers éléments, avantageusement le connecteur de l'instrument médical étant maintenu par l'un des deuxièmes éléments.

L'invention apparaîtra plus clairement à la lecture de la description qui va suivre, donnée uniquement à titre d'exemple non limitatif, et faite en référence aux dessins dans lesquels :
Les figures 1 et 2 sont des vues schématiques en perspective d'un exemple d'une enceinte de désinfection selon l'invention ;
La figure 3 est une vue schématique en perspective d'un exemple de deux éléments du support selon l'invention ; et
La figure 4 est une vue schématique d'un exemple d'instrument médical propre à être désinfecté par l'enceinte de désinfection de la figure 1.

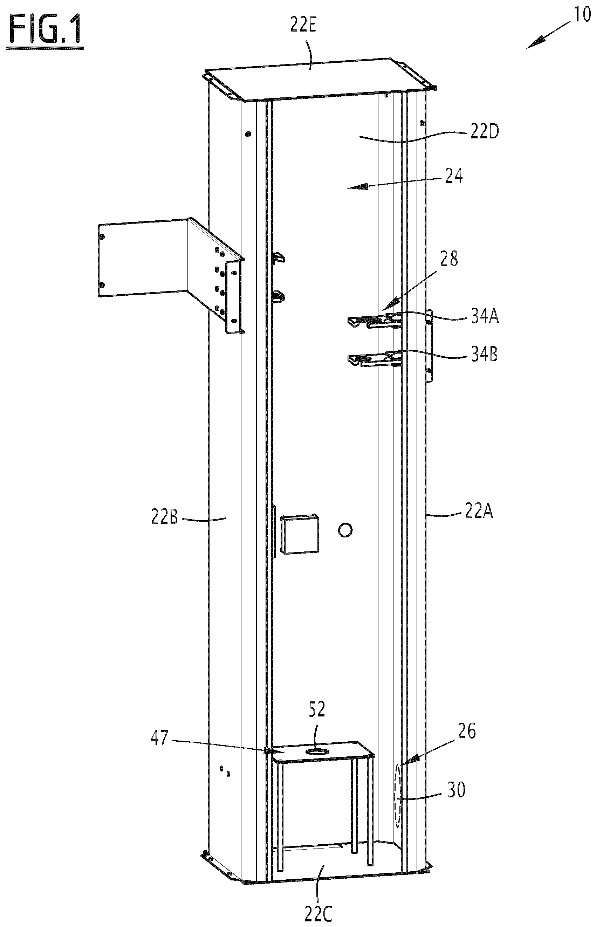

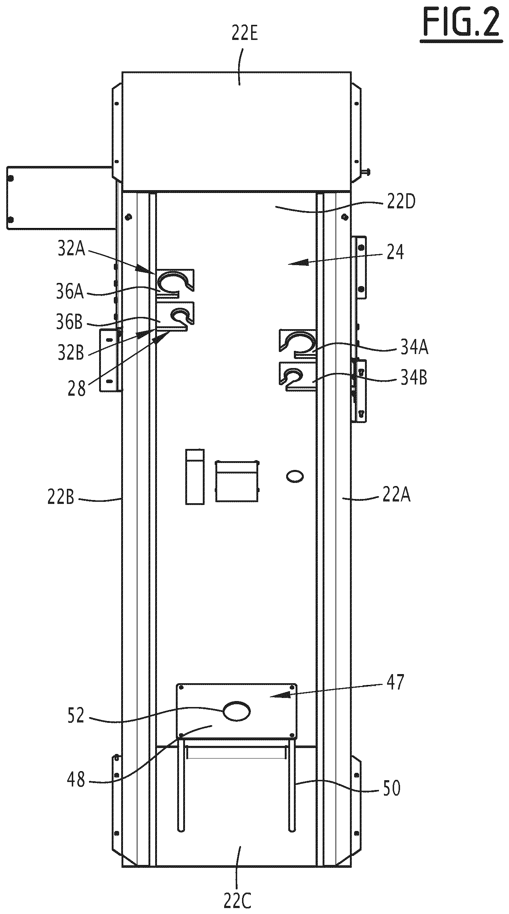

Les figures 1 et 2 illustrent un exemple d'une enceinte de désinfection 10 selon l'invention. L'enceinte de désinfection 10 est configurée pour désinfecter en entier un instrument médical 12.

Par désinfection en entier de l'instrument médical 12, on entend une désinfection de toutes les parties de l'instrument médical 12 dans l'enceinte de désinfection 10.

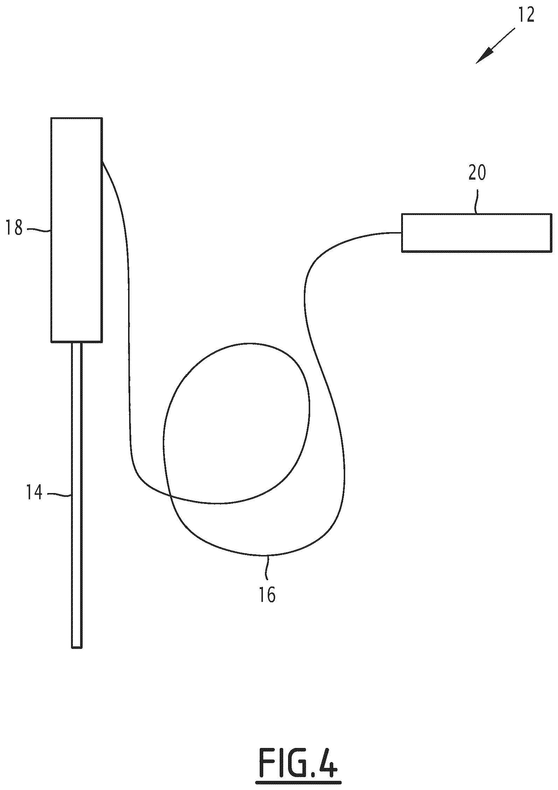

La figure 4 illustre un exemple d'un instrument médical 12 propre à être désinfecté par l'enceinte de désinfection 10.

L'instrument médical 12 comprend par exemple une partie active 14. La partie active 14 est par exemple un tube destiné à être inséré dans le patient, par exemple dans une narine du patient.

De préférence, l'instrument médical 12 comprend une poignée 18 connectée à la partie active 14. La poignée 18 est avantageusement fixée à une extrémité de la partie active 14 et est destinée à être saisie par un utilisateur afin de faciliter l'insertion de la partie active 14 dans le patient.

L'instrument médical 12 comprend, en outre, de préférence une partie de raccordement 16 et de manière encore plus préférentielle également un connecteur 20.

La partie de raccordement 16 est par exemple un cable de raccordement reliant la partie active 14 au connecteur 20.

Le connecteur 20 est par exemple configuré pour être connecté à une unité de mesure et/ou d'affichage, de sorte notamment à collecter et/ou afficher des informations captées par la partie active 14.

L'instrument médical 12 est par exemple une sonde médicale, notamment une sonde médicale échographique, et plus particulièrement une sonde médicale de type « échographie transoephagienne » (ETO) ou une sonde médicale endoscopique, par exemple de type « oto-rhino-laryngologie » (ORL). La sonde médicale est par exemple une sonde pédiatrique ou pour adulte.

L'enceinte de désinfection 10 comprend un volume de désinfection 24 délimité par au moins une paroi, un dispositif de génération de rayonnements UV-C 26 et un support 28.

Avantageusement, l'au moins une paroi comprend un fond 22C.

De préférence, l'au moins une paroi comprend au moins une première zone et une deuxième zone différente de la première zone.

Dans un mode de réalisation préférentielle, l'enceinte de désinfection 10 comprend une pluralité de parois 22A, 22B, 22C, 22D, 22E définissant entre elles le volume de désinfection 24. Plus particulièrement, les parois 22A, 22B, 22C, 22D, 22E, 22F définissent entre elles un volume de désinfection 24 fermé, par exemple de forme parallélépipédique.

Dans le mode de réalisation de l'invention illustré sur les figures 1 et 2, la pluralité de parois comprend par exemple une première paroi latérale 22A et une deuxième paroi latérale 22B opposées l'une de l'autre, un fond 22C et un capot 22E opposé au fond 22C et également une base 22D définissant une troisième paroi latérale et une porte (non représentée) en regard de la base 22D.

Par exemple, la première paroi latérale 22A définit la première zone et la deuxième paroi latérale 22B définit la deuxième zone.

En particulier, le capot 22E et la fond 22C sont par exemple perpendiculaires aux deux parois latérales 22A, 22B. De même, la base 22D et la porte sont par exemple perpendiculaires aux deux parois latérales 22A, 22B, au capot 22E et au fond 22C.

Dans une variante non-illustrée, l'enceinte de désinfection 10 comprend une unique paroi 22 délimitant le volume de désinfection 24, qui est par exemple cylindrique ou sphérique.

Le dispositif 26 de génération de rayonnements UV-C est configuré pour générer des rayonnements UV-C dans le volume de désinfection 24.

Par exemple, le dispositif 26 de génération de rayonnements UV-C comprend une pluralité de sources de rayonnements UV-C 30 disposées à l'intérieur de l'enceinte de désinfection 10, et par exemple sur au moins l'une des parois 22, par exemple au niveau d'au moins un des bords latéraux de la base 22D comme illustré sur la figure 1.

Chaque source 30 de rayonnements UV-C est par exemple une lampe à mercure générant des rayonnements UV-C. En particulier, chaque lampe à mercure 30 est apte à utiliser du mercure pour exciter un gaz et produire des rayonnements ultraviolets UV-C.

Par rayonnements UV-C, on entend des rayonnements caractérisés par une longueur d'onde comprise entre 200 et 280 nm

Alternativement, chaque source 30 de rayonnements UV-C est une lampe à LED (Light-Emitting Diode se traduisant en diode électroluminescente).

Le support 28 est configuré pour maintenir l'instrument médical 12 en entier à l'intérieur du volume de désinfection 24.

Autrement dit, lorsque l'instrument médical 12 est maintenu par le support 28, l'ensemble des éléments constituant l'instrument médical 12 sont disposés à l'intérieur du volume de désinfection.

En particulier, la partie active 14, la partie de raccordement 16, la poignée 18 et le connecteur 20 sont entièrement maintenus à l'intérieur du volume de désinfection 24.

Plus particulièrement, les deux extrémités de la partie de raccordement 16 se trouvent à l'intérieur du volume de désinfection 24, lorsque l'instrument médical 12 est maintenu par le support 28.

Le support 28 est transparent aux rayonnements UV-C.

En particulier, le support 28 a une transmittance aux rayonnements UV-C supérieure à 30%, et de préférence supérieure à 50%, et de manière encore plus préférentielle supérieure à 90%.

Par « transmittance », on entend le rapport des rayonnements transmis par le support 28 sur les rayonnements incidents sur le support 28.

Autrement dit, au moins 30% des rayonnements UV-C incidents sur le support 28 sont transmis à travers le support 28.

Le support 28 est préférablement fabriqué en quartz, de préférence du quartz synthétique, tel que par exemple un verre de quartz optique de catégorie JGS1.

Alternativement, le support 28 est fabriqué en un polymère, préférablement un copolymère d'oléfine cyclique (COC de l'anglais « Cyclic olefin copolymer »), en particulier du TOPAS^{®} 8007X10, ou un polyméthylpentène, tel que le TPX^{™} RT18, ou de l'éthylène-propylène fluoré.

Comme illustré sur les figures 1 et 2, le support 28 comprend au moins une paire d'éléments 32A, 32B, et de préférence deux paires d'éléments 32A, 32B.

Chaque paire d'éléments 32A, 32B comprend un premier élément 34A, 34B et un deuxième élément 36A, 36B.

Chaque premier élément 34A, 34B et deuxième élément 36A, 36B est configuré pour maintenir une portion de l'instrument médical 12, par exemple la poignée 18 ou le connecteur 20.

Le premier élément 34A, 34B et le deuxième élément 36A, 36B d'une même paire 32A, 32B sont de préférence identiques.

Chaque premier élément 34A, 34B est par exemple fixé à la première paroi latérale 22A.

Chaque deuxième élément 36A, 36B est par exemple fixé à la deuxième paroi latérale 22B, de préférence à une hauteur par rapport au fond 22C différente du premier élément 34A, 34B de la même paire.

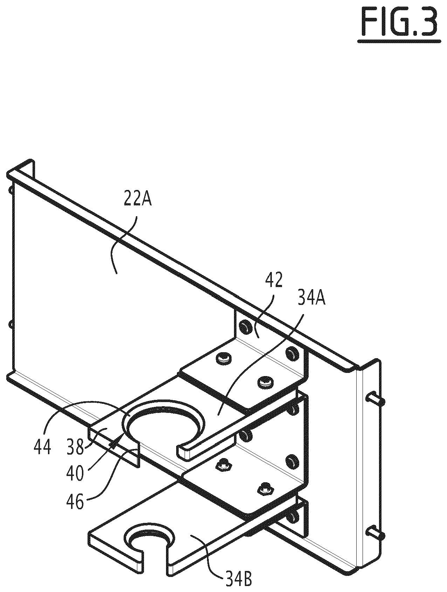

Comme illustré sur la figure 3, chaque premier élément 34A, 34B et/ou chaque deuxième élément 36A, 36B comprend une plaque 38 comprenant une ouverture latérale d'insertion 40 de sorte à permettre l'insertion d'une portion de l'instrument médical 12.

En particulier, la plaque 38 est par exemple une plaque réalisée dans un matériau transparent aux rayonnements UV-C, par exemple en quartz.

Dans l'exemple illustré sur la figure 3, la plaque 38 est de forme sensiblement rectangulaire.

Par exemple, la plaque 38 a une épaisseur comprise entre 0,5 cm et 2 cm.

Comme cela est visible sur la figure 3, la plaque 38 est par exemple fixée à la paroi latérale 22A, 22B correspondante par des équerres de fixations 42 fixées par exemple par des vis.

L'ouverture latérale d'insertion 40 est de préférence ménagée sur la plaque 38 de sorte à permettre l'insertion d'une portion de l'instrument médical 12 et le maintien de celle-ci.

L'ouverture latérale d'insertion 40 comprend par exemple un perçage circulaire 44 et un dégagement 46.

Le perçage circulaire 44 présente, de préférence, un diamètre adapté à maintenir une portion de l'instrument médical 12, comme par exemple la poignée 18 et/ou le connecteur 20. Le perçage circulaire 44 présente par exemple un diamètre compris entre 20 mm et 50 mm.

Comme cela est visible sur la figure 3, le dégagement 46 est de préférence ménagé de sorte à relier un bord de la plaque 38 au perçage circulaire 44, afin de permettre l'insertion de la portion de l'instrument médical 12 dans le perçage circulaire 44.

Le dégagement 46 est avantageusement orienté d'un angle d'environ 45° par rapport aux bords de la plaque 38.

Avantageusement, l'ouverture latérale d'insertion 40 du premier élément 34A, 34B et du deuxième élément 36A, 36B d'une même paire d'éléments 32A, 32B sont identiques.

De préférence, dans le mode de réalisation dans lequel le support 28 comprend deux paires d'éléments 32A, 32B, les ouvertures latérales d'insertion 40 des premiers éléments 34A 34B, respectivement des deuxièmes éléments 36A, 36B, des deux paires sont différentes, et en particulier ont des dimensions différentes. Une telle différence permet une plus grande adaptabilité et notamment un maintien d'instruments médicaux 12 avec des dimensions et des configurations variées.

Par ailleurs, le support 28 comprend de préférence un troisième élément 47 fixé au fond 22C.

Le troisième élément 47 est de préférence adapté pour maintenir une portion de l'instrument médical 12, et en particulier la partie active 14 de l'instrument médical 12, à distance des parois 22 de l'enceinte de désinfection 10.

Le troisième élément 47 comprend par exemple une plaque 48 fixée au fond 22C par l'intermédiaire de pieds de fixation 50.

Plus particulièrement, la plaque 48 est sensiblement parallèle au fond 22C et est maintenue à distance de celui-ci par les pieds de fixation 50.

La plaque 48 est par exemple une plaque réalisée dans un matériau transparent aux rayonnements UV-C, par exemple en quartz.

Avantageusement, la plaque 48 comprend un perçage circulaire 52 avantageusement de diamètre adapté pour recevoir la partie activé 14 de l'instrument médical 12. Le diamètre est par exemple compris entre 30 mm et 50 mm.

Un procédé de désinfection de l'instrument médical 12 dans l'enceinte de désinfection 10 va maintenant être décrit.

Premièrement, une enceinte de désinfection 10 telle que décrite ci-dessus est fournie.

Ensuite, l'instrument médical 12 destiné à être désinfecté est positionné dans le volume de désinfection 24 de l'enceinte de désinfection 10.

En particulier, l'instrument médical 12 est positionné en entier à l'intérieur du volume de désinfection 24, l'instrument médical 12 étant maintenu par le support 28.

En particulier, l'opérateur insère une portion de l'instrument médical 12 dans le perçage circulaire 44 de chacun d'au moins l'un des premiers éléments 34A, 34B et d'au moins l'un des deuxièmes éléments 36A, 36B.

Plus précisément, dans le mode de réalisation préféré et illustré sur les figures, la poignée 18 de l'instrument médical 12 est par exemple maintenue par l'un des premiers éléments 34A, 34B et le connecteur 20 de l'instrument médical 12 est maintenu par l'un des deuxièmes éléments 36A, 36B. La partie de raccordement 16 s'étend entre ces deux éléments.

En fonction de l'instrument médical 12 à désinfecter, le premier élément 34A maintenant la poignée 18 et le deuxième élément 36A maintenant le connecteur 20 appartiennent à la même paire d'éléments ou à des paires différentes.

La partie active 14 est de préférence maintenue dans le troisième élément 47 du support 28. En particulier, la partie active 34 est insérée dans le perçage circulaire 52 ménagée dans la plaque 48 du troisième élément 47.

La porte (non représentée) est ensuite fermée.

Un cycle de désinfection par rayonnements UV-C générée par le dispositif 26 de génération de rayonnements UV-C est ensuite mis en œuvre.

Une telle enceinte de désinfection 10 est particulièrement avantageuse pour la désinfection d'instruments médicaux 12, et en particulier de sondes médicales.

En particulier, grâce au support 28, l'instrument médical 12 est maintenu en entier dans le volume de désinfection 12, ce qui évite qu'une portion de l'instrument ne soit pas désinfectée.

Par ailleurs, la caractéristique selon laquelle le support 28 est transparent aux rayonnements UV-C garantit que même les portions de l'instrument médical 12 qui sont directement maintenues par le support 28, notamment qui sont en contact avec celui-ci, soient atteintes par les rayonnements UV-C, et donc désinfectées.

En outre, le mode de réalisation utilisant un support 28 en quartz est particulièrement avantageux, car ce matériau garantit une transmittance aux rayonnements UV-C supérieure à 90%. Par ailleurs, un tel matériau a une très bonne robustesse mécanique et ses capacités de transmission des rayonnements UV-C restent inchangées même après des centaines d'heures d'exposition.

Par ailleurs, le support 28 est adapté pour maintenir différents types et tailles d'instruments médicaux 12.

L'homme du métier comprendra que les modes de réalisation et variantes précédemment décrits peuvent être combinés entre eux pourvu qu'ils soient compatibles techniquement.

## Revendications

1. Enceinte de désinfection (10) d'un instrument médical (12) comprenant :
- un volume de désinfection (24) délimité par au moins une paroi (22A-E),
- un dispositif de génération de rayonnements UV-C (26) configuré pour générer des rayonnements UV-C dans le volume de désinfection (24), et
- un support (28) configuré pour maintenir l'instrument médical (12) en entier à l'intérieur du volume de désinfection (24), le support (28) étant transparent aux rayonnements UV-C.

2. Enceinte (10) selon la revendication 1, dans laquelle le support (28) a une transmittance aux rayonnements UV-C supérieure à 30%, et de préférence supérieure à 50%.

3. Enceinte (10) selon la revendication 1 ou 2, dans laquelle le support (28) comprend au moins une paire d'éléments (32A, 32B) comprenant un premier élément (34A, 34B) et un deuxième élément (36A, 36B), l'instrument médical (12) étant destiné à être maintenu par le premier (34A, 34B) et le deuxième (36A, 36B) éléments.

4. Enceinte (10) selon la revendication 3, dans laquelle l'au moins une paroi (22A-E) comprend au moins une première zone (22A) et une deuxième zone (22B) différente de la première zone (22A), le premier élément (34A, 34B) étant fixé à la première zone et le deuxième élément (36A, 36B) étant fixé à la deuxième zone (22B).

5. Enceinte (10) selon la revendication 3 ou 4, dans laquelle le support (28) comprend deux paires d'éléments (32A, 32B).

6. Enceinte (10) selon l'une quelconque des revendications 3 à 5, dans laquelle le premier (34A, 34B) et/ou le deuxième (36A, 36B) élément du ou de chaque paire d'éléments (32A, 32B) comprend une plaque (38) comprenant une ouverture latérale d'insertion (40) de sorte à permettre l'insertion d'une portion de l'instrument médical (12).

7. Enceinte (10) selon l'une quelconque des revendications précédentes, dans laquelle l'au moins une paroi (22A-E) comprend un fond (22C), le support (28) comprenant un troisième élément (47) fixé au fond (22C), l'instrument médical (12) étant destiné à être maintenu par le troisième élément (47).

8. Enceinte (10) selon l'une quelconque des revendications précédentes, dans laquelle le support (28) est fabriqué en quartz, de préférence du quartz synthétique.

9. Procédé de désinfection d'un instrument médical (12) comprenant les étapes suivantes :
a. fourniture d'une enceinte (10) selon l'une quelconque des revendications précédentes, et
b. positionnement de l'instrument médical (12) en entier à l'intérieur du volume de désinfection (24), l'instrument médical (12) étant maintenu par le support (28).

10. Procédé selon la revendication 9, dans laquelle l'instrument médical (12) comprend une poignée (18) et de préférence au moins un connecteur (20), l'enceinte de désinfection (10) étant selon l'une quelconque des revendications 2 à 8, la poignée (18) de l'instrument médical (12) étant maintenue par l'un des premiers éléments (34A, 34B), avantageusement le connecteur (20) de l'instrument médical (12) étant maintenu par l'un des deuxièmes éléments (36A, 36B).
